# EUROPEAN PATENT APPLICATION

(11) **EP 1 162 274 A1**
(43) Date of publication of application: **12.12.2001**
(21) Application number: 00112345.4
(22) Date of filing: 09.06.2000
(51) Int. Cl.: C12Q 1/48, G01N 33/68

(54) **Method for identifying compounds modifying methyltransferase-dependent chromosome stability**

(71) Applicant: Boehringer Ingelheim International GmbH, 55218 Ingelheim am Rhein (DE)
(72) Inventor: Jenuwein, Thomas, Dr., 1030 Wien (AT); Rea, Stephen, Dr., Galway (IE); Eisenhaber, Frank, Dr., 1210 Wien (AT); O'Carroll, Donal, Dr., Wicklow (IE)
(74) Representative: Laudien, Dieter, Dr.

(57) **Abstract**

A method for identifying compounds that alter higher order chromatin dependent chromosome stability is based on determining the compounds' ability to modify a methyltransferase with Suv39h-like methyltransferase activity. The identified compounds are useful in therapy, in particular the therapy of human cancer.

## Description

The invention relates to a method for identifying compounds influencing chromosome dynamics in eukaryotic cells. In particular, the invention relates to the treatment and prevention of human conditions by modulating higher order chromatin dependent chromosome stability.

Higher-order chromatin is essential for epigenetic gene control and for the functional organisation of chromosomes. Differences in higher-order chromatin structure have been linked with distinct covalent modifications of histone tails which regulate transcriptional 'on' or 'off' states (Grunstein, 1998; Turner, 1998; Strahl and Allis, 2000) and influence chromosome condensation and segregation (Karpen and Allshire, 1997; Wei *et al*., 1999).

Histones constitute a highly conserved family of proteins (H3, H4, H2A, H2B, H1) which are the major components of eukaryotic chromatin structure. Histones compact genomic DNA into basic repeating structural units, the nucleosomes. In addition to their DNA packaging function, histones have been proven to be integral components of the molecular machinery that regulates gene expression.

Post-translational modifications of histone N-termini, particularly of H4 and H3, are well documented and have functionally been characterised as changes in acetylation (Grunstein, 1998; Turner, 1998; Strahl and Allis, 2000), phosphorylation (Wei *et al*., 1999) and, most recently, methylation (Chen *et al*., 1999; Strahl *et al*., 1999). In contrast to the large number of described histone acetyltransferases (HATs) and histone deacetylases (HDACs), genes encoding enzymatic activities that regulate phosphorylation (Sassone-Corsi *et al*., 1999) or methylation (Chen *et al*., 1999) of histone N-termini are only beginning to be identified. Moreover, the interdependence of the different histone tail modifications for the integration of transcriptional output or higher-order chromatin organisation is currently not understood.

Overall, there is increasing evidence that the regulation of normal and aberrant cellular proliferation is not only affected on the transcriptional level, but that also a higher level of regulation is involved, i.e. the organisation of chromatin structure through the modification of histone molecules. The determination of the proteins and the molecular mechanisms involved in histone modification will contribute to the understanding of the cellular proliferation program and will thus shed led light on the mechanisms involved in aberrant proliferation occurring in tumour formation and progression (Jacobson and Pillus, 1999).

Genetic screens for suppressors of position effect variegation (PEV) in *Drosophila* (Reuter and Spierer, 1992) and *S.pombe* (Allshire *et al*., 1995) have identified a subfamily of approximately 30-40 loci which are referred to as *Su(var)*-group (Wallrath, 1998) genes. Interestingly, several histone deacetylases (De Rubertis *et al*., 1996), protein phosphatase type 1 (Baksa *et al*., 1993) and S-adenosyl methionine synthetase (Larsson *et al*., 1996) have been classified as *Su(var)s.* In contrast, *Su(var)2-5* (which is allelic to *HP1*) (Eissenberg *et al*., 1992), *Su(var)3-7* (Cléard *et al*., 1997) and *Su(var)3-9* (Tschiersch *et al*., 1994; Schotta and Reuter, 2000) encode heterochromatin-associated proteins. *Su(var)* gene function thus suggests a model, in which modifications at the nucleosomal level may initiate the formation of defined chromosomal subdomains that are then stabilised and propagated by heterochromatic SU(VAR) proteins (Henikoff, 1997).

*Su(var)3-9* is dominant over most PEV modifier mutations (Tschiersch et *al*., 1994), and mutants in the corresponding *S.pombe clr4* gene (Ivanova *et al*., 1998) disrupt heterochromatin association of other modifying factors and result in chromosome segregation defects (Ekwall *et al*., 1996). Recently, human (*SUV39H1*) and murine (*Suv39h1* and *Suv39h2) Su(var)3-9* homologues have been isolated (Aagaard *et al*., 1999). It has been shown that they encode heterochromatic proteins which associate with mammalian HP1 (Aagaard *et al*., 1999). The SU(VAR)3-9 protein family combines two of the most evolutionarily conserved domains of 'chromatin regulators': the chromo (Aasland and Stewart, 1995; Koonin *et al*., 1995) and the SET (Tschiersch *et al*., 1994; Jenuwein *et al*., 1998) domain. Whereas the 60 amino acids chromo domain represents an ancient histone-like fold (Ball et *al*., 1997) that directs eu- or heterochromatic localisations (Platero *et al*., 1995), the molecular role of the 130 amino acids SET domain has remained enigmatic. Overexpression studies with human SUV39H1 mutants indicated a dominant interference with higher-order chromatin organisation that, surprisingly, suggested a functional relationship between the SET domain and the distribution of phosphorylated (at serine 10) H3 (Melcher *et al*., 2000). The experiments of the present invention show that mammalian SU(VAR)3-9 related proteins (human SUV39H1, murine Suv39h1 and murine Suv39h2) are SET domain-dependent H3-specific histone methyltransferases which selectively methylate lysine 9 ("K9") of the H3 N-terminus. Methylation of K9 negatively regulates phosphorylation of serine 10 and reveals a 'histone code' that appears intrinsically linked to the organisation of higher-order chromatin. (In the following, histone methyltransferases are termed "HMTases" or, more generally, "MTases").

It was an object of the invention to gain further insight in the molecular pathways leading to histone modifications and higher-order chromatin organisation in order to harness these findings as a target for interfering with aberrant gene expression and genomic instability through chromosome missegregation and thus provide new cancer therapies.

In particular, it was an object of the invention to investigate the function of members of the SU(VAR)3-9 protein family with the view to develop novel strategies to affect higher-order chromatin dependent chromosome stability. Such strategies can be employed in therapies for the treatment of conditions in which aberrant gene expression and genomic instability through chromosome missegregation are causally involved. (The term "chromosome stability" implies successful segregation of chromosomes resulting in the maintenance of a stable karyotype).

To solve the problem underlying the present invention, in a first step bioinformatic techniques were applied. Using the SET domains of the SU(VAR)3-9 protein family as a starting alignment, significant sequence and secondary structure similarities (see Methods) to six plant protein methyltransferases were detected.

To investigate whether the SET domain of human SUV39H1 has enzymatic activity, histones were tested as possible substrates for *in vitro* methylation. The obtained results demonstrate that SUV39H1 harbours an intrinsic histone methyltransferase activity and suggest that this HMTase activity resides in the C-terminal SET domain.

Using recombinant proteins, both murine GST-Suv39h1(82-412) and the corresponding human SUV39H1 fusion protein [GST-SUV39H1(82-412)] were shown to be catalytically active. Short internal deletions were introduced into the two conserved regions of the SET domain core in GST-SUV39H11(82-412), and additional mutants lacking the C-terminal tail (ΔC-tail) or the SET-associated cysteine-rich region (Δcys) were generated. All mutant proteins failed to demonstrate HMTase activity.

Although these results suggest a significant contribution by the cysteine-rich regions, their apparent absence in the plant methyltransferases does not prevent catalytic activity. To investigate enzyme function of the SET domain in more detail, point mutations were introduced into the most highly conserved motif. *In vitro* HMTase assays indicated that all point mutations, with the exception of one, abolished enzymatic activity. Surprisingly, the latter mutation resulted in an hyperactive enzyme with approximately 20-fold increased activity. The data obtained define the ₃₂₀HφφNHSC₃₂₆ motif in the SET domain as an important catalytic site.

Because the SET domain is one of the most conserved protein motifs in chromatin regulators (Stassen *et al*., 1995; Jenuwein *et al*., 1998), it was next analysed whether SU(VAR)3-9 family members or other SET domain proteins contain HMTase activity. GST-fusion products of the extended SET domains of *S.pombe* CLR4 (Ivanova *et al*., 1998), human EZH2 (Laible *et al*., 1997) and human HRX (Tkachuk *et al*., 1992) were generated that would correspond to GST-SUV39H1 (82-412). Interestingly,
GST-CLR4(127-490) displayed pronounced HMTase activity at three- to five-fold increased levels as compared to the recombinant SUV39H1 product, consistent with CLR4 carrying an arginine at the hyperactive position. The results obtained from this analysis show, in agreement with the mutational analysis of SUV39H1, that HMTase activity towards free histones appears to require the combination of the SET domain with adjacent cysteine-rich regions, which is a quality found in only a restricted number of SET domain containing proteins.

These experiments indicated that the HMTase activity of mammalian SU(VAR)3-9 related proteins is selective for histone H3 under the chosen assay conditions. To examine this finding in more detail, *in vitro* methylation reactions were performed with individual histones. It could be shown that H3 is specifically methylated by GST-Suv39h1(82-412), whereas no signals are detected with H2A, H2B or H4. Methylation of H3 has been shown to occur predominantly at lysine 4 in a wide range of organisms, as well as at lysine 9 in HeLa cells, although the responsible HMTase(s) have yet to be defined (Strahl *et al*., 1999). To investigate the site utilisation profile of Suv39h1, unmodified peptides comprising the wild-type H3 N-terminus and a mutant K9L peptide were tested as substrates. Additionally, insulin and peptides comprising the N-termini of CENP-A (Sullivan *et al*., 1994), macroH2A (Pehrson and Fried, 1992) were included. These *in vitro* assays revealed selective methylation of the wild-type H3 peptide. The data obtained also suggested that the H3 N-terminus is a preferred residue for Suv39h1-dependent HMTase activity.

To more definitively determine this site preference, the wild-type H3 N-terminal peptide was *in vitro* methylated by GST-Suv39h1 (82-412), using S-adenosyl-[*methyl*-³H]-L-methionine. The labelled peptide, purified by reverse-phase HPLC, was then directly microsequenced, and
³H-incorporation associated with each individual amino acid was analysed. The results confirmed selective transfer of methyl-label to lysine 9, demonstrating that Suv39h1 is a highly site-specific HMTase for the H3 N-terminus *in vitro.*

Murine *Suv39h* genes are encoded by 2 loci, *Suv39h1* and *Suv39h2.* To investigate the *in vivo* significance of *Suv39h* function and *Suv39h* dependent K9 H3 methylation, mouse strains deficient for both *Suv39h1* and *Suv39h2* were generated. *Suv39h1* and *Suv39h2* deficient strains were intercrossed to produce *Suv39h* double deficient mice. Double mutant mice were born in sub-Mendelian ratios. Some double null embryos exhibited severe growth retardation and exencephaly. In addition surviving double mutants were growth retarded, suggesting a role for *Suv39h* in cell proliferation.

In order to determine whether the embryonic phenotypes in *Suv39h* null mice can be attributed to mitotic defects, PMEFs (primary mouse embryonic fibroblasts) derived from *Suv39h* double mice were analysed. *Suv39h* double null PMEFs display a reduced G1-index and an increased proportion of cells with aberrant nuclear morphologies, reminiscent of division defects during mitosis. Furthermore, double null cells also show genomic instabilities and readily become aneuploid. The severity of these aneuploidies increases with higher passage numbers. The inability of *Suv39h* double null cells to maintain a stable karyotype may underlie the *Suv39h* embryonic phenotype.

Phosphorylation at serine 10 in the N-terminal tail of H3 (phosH3) has been shown to be required for condensation and subsequent segregation of chromosomes (Wei *et al*., 1999). During the cell cycle, phosH3 initiates within pericentric heterochromatin in late G2 and then progresses along the entire chromosomes during mitosis (Hendzel *et al*., 1997). It was found that in wild-type PMEFs, approximately 7% of the cells stain positive for the characteristic, heterochromatin-associated phosH3 foci. In contrast, this number is increased by a factor of about 3-fold in *Suv39h* double null PMEFs. This result suggested that the overall levels of phosH3 may be enhanced in *Suv39h* double null PMEFs. This was confirmed biochemically. Together, the obtained data are most consistent with a model in which Suv39h-mediated methylation of lysine 9 in H3 negatively regulates phosphorylation of serine 10.

Together, these data clearly demonstrate crucial roles for *Suv39h* during cell division. Loss of *Suv39h* function impairs K9 histone H3 metylation and induces defective cell division resulting in genome instabilities. Segregation defects/genome instability underlies the aetiology of many human cancers (Lengauer *et al*., 1997) and are often a prerequisite for tumour progression. These observations make *Suv39h* an excellent candidate for novel therapeutic approaches for tumour therapies.

The results obtained in the experiments of the present invention show that members of the SU(VAR)3-9 protein family have HMTase activity which identifies them as novel targets for the therapy of proliferative disorders, in particular cancer.

The identification of members of the SU(VAR)3-9 protein family, exemplified by human SUV39H1, murine Suv39h1 and murine Suv39h2, as K9 specific histone H3 MTases is the prerequisite for designing assay methods that allow for finding compounds altering, in particular interfering with, higher order chromatin dependent chromosome stability, which is the basis for novel therapeutic approaches.

The present invention relates to a method for identifying a compound that alters higher order chromatin dependent chromosome stability, said method comprising incubating a substrate for a methyltransferase, in the presence of a methyl donor, with a MTase with Suv39h-like MTase activity, in the presence or absence of a test compound and determining whether the compound modulates the MTase activity.

The group of MTases with Suv39h-like MTase activity (in the following also termed "Suv39h-like MTases") encompasses enzymes which display K9 histone H3 MTase activity or methyltransferase activity for other yet to be identified substrates.

The term "K9 histone H3" is not limited to the human SUV39H or mouse Suv39h substrate (i.e. the methylation site at lysine 9 of histone H3), but is meant to encompass any substrate of the histone or histone variant-type of protein the methylation of which results in the below-defined epigenetic signal.

Additional members of the group of MTases can be identified by bioinformatic/biochemical techniques and tested biochemically. By way of example, in a first step, searching data bases for similarities, as described in Example 1. Next, an identified candidate can be verified as a MTase with Suv39h-like MTase activity in biochemical assays similar to or identical with those described in the Examples.

This group of Suv39h-like MTases also encompasses MTases with specificities for other histone H3 residues than K9 or for substrates other than histone H3, which are, like the Suv39h K9 histone H3 HMTase activity observed in the present invention, required for higher order chromatin dependent chromosome stability. This epigenetic signal may be a consequence of histone methylation at lysine 9 on H3 alone; however, it cannot be excluded that MTase activity on undefined substrates or a combination of substrate methylation and other covalent modifications, such as phosphorylation or acetylation, at other histone residues are involved.

In the experiments of the present invention, Suv39h variants with point mutations in the SET domain were shown to confer hyperactive HMTase activity to the protein, these Suv39h variants may be advantageously used in the method of the invention.

In a preferred embodiment, the MTase is mouse Suv39h1 or Suv39h2, most preferably, the MTase is human SUV39H1 or SUV39H2.

Since it has been shown in the present invention that recombinant Suv39h retains HMTase activity, most preferably, a recombinant MTase is employed. Suv39h or Suv39h variants can be produced recombinantly according to standard methods by expression in suitable hosts, e.g. bacteria, yeast, insect or eucaryotic cells and purified, e.g. on glutathioneagarose columns if it has been tagged with GST.

The Suv39h1 and SUV39H1 cDNA sequences are known from the literature (Aagaard et al., 1999), the Suv39h2 cDNA sequence is shown in SEQ ID NO: 1; the human SUV39H2 cDNA is defined by the ESTs as shown in SEQ ID NO: 3 - 6.

In the case of testing the compounds for their effect on Suv39h activity, the assay comprises, as its essential features, incubating a histone H3 protein or histone H3 N-terminal fragment including K9, a methyl donor, S-adenosyl-L-Methionine with a preparation containing a Suv39h MTase activity and determining MTase activity in the presence or absence of a test substance.

MTase substrates useful in the method of the invention may be those equivalent to or mimicking the naturally occurring substrates, e.g. biochemically purified histone H3, recombinantly produced histone H3, or a histone H3 peptide that contains the K9 methylation site, or other yet to be identified proteins which act as substrates for Suv39h MTases. Novel Suv39h substrates can be identified by bioinformatic and biochemical techniques and tested using the biochemical assays described in the Examples of the present invention. For example, novel Suv39h substrates can be identified by co-immunoprecipitation techniques. Suv39h proteins or tagged versions of Suv39h proteins can be immunoprecipitated with specific antisera and interacting proteins identified by mass spectroscopy techniques. A yeast two hybrid screen using Suv39h proteins or portions of Suv39h proteins as a bait can also be employed to identify novel interacting protein from a variety of cDNA libraries.

In a preferred embodiment, the histone H3 fragment
ARTKQTARKSTGGKAPRKQL (SEQ ID NO:7) is employed. Alternatively, a modified peptide may be used for which the MTase has increased affinity/activity. Such peptides can be designed by exchanging and/or adding and/or deleting amino acids and testing the substrate in serial experiments for MTase affinity/activity.

The methyl donor preferably carries a detectable label, e.g. a radioactive or a chromogenic label, which can be quantified upon transfer to the substrate.

Preferably, the methyl donor is radio-labelled methionine or S-adenosyl-L-methionine.

Alternatively to using a labelled methyl donor, the substrate, upon methylation by the enzyme, is used to serve as an epitope which can be recognised by a specific antibody and hence be quantified by standard immunoassay techniques, e.g. ELISAs. Antibodies useful in this type of assay can be obtained by using the methylated substrate, preferably a small peptide, e.g. the peptide with the sequence shown in SEQ ID NO:7, as an antigen and obtaining polyclonal or monoclonal antibodies according to standard techniques.

For small scale applications, the screening method can be based on an assay as described in Example 2, 3 or 4.

In a preferred embodiment, the method of the invention is performed on a high-throughput scale. For this embodiment, the major assay components, in particular Suv39h, are employed in recombinant form.

For the high throughput format, the screening methods of the invention to identify MTase inhibitors, are carried out according to standard assay procedures. Such assays are based on the catalytic transfer, mediated by Suv39h or a Suv39h variant, of a methyl group from a chromogenic substrate to a histone H3 peptide. To achieve this, the substrate, e.g. histone H3 or a variant or fragment thereof, is immobilised on a carrier, usually a microtiter plate, and incubated with recombinant Suv39h or a Suv39h variant and a methyl donor. The methyl donor preferably carries a chromogenic or radioactive label. Upon transfer of the methyl group to the substrate by Suv39h, in the case of a chromogenic reagent, the methyl donor changes colour which can be quantified. In the case of using a radioactive methyl donor, the methyl group is transferred to the substrate and can be quantified.

Alternatively, the methylation of the substrate can be quantified by ELISA using an antibody specific for the methylated substrate. If a test substance is an inhibitor of the MTase activity, there will be, depending on the detection system and depending on whether the test substance has an inhibiting or an activating effect, a decrease or an increase in the detectable signal.

In the high-throughput format, compounds with a modulating effect Suv39h MTase activity can be identified by screening test substances from compound libraries according to known assay principles, e.g. in an automated system on microtiter plates.

By providing a method to identify compounds which exert their effect by directly modulating, in particular by inhibiting, a Suv39h-like MTase, the present invention provides a basis for inhibiting the proliferation of rapidly dividing animal cells, in particular tumour cells.

The compounds identified in the above methods, which are also subject of the invention, have the ability to interfere with chromosome stability and high fidelity chromosome segregation by modulating the MTase activity of Suv39h.

In a preferred embodiment, the compounds of the invention are inhibitors of Suv39h HMTase activity.

Preferably, the compounds are specific modulators of Suv39h.

The present invention also relates to compounds, which act as modulators of a Suv39h-like MTase activity, in particular modulators of Suv39h, for use in human therapy, in particular cancer therapy.

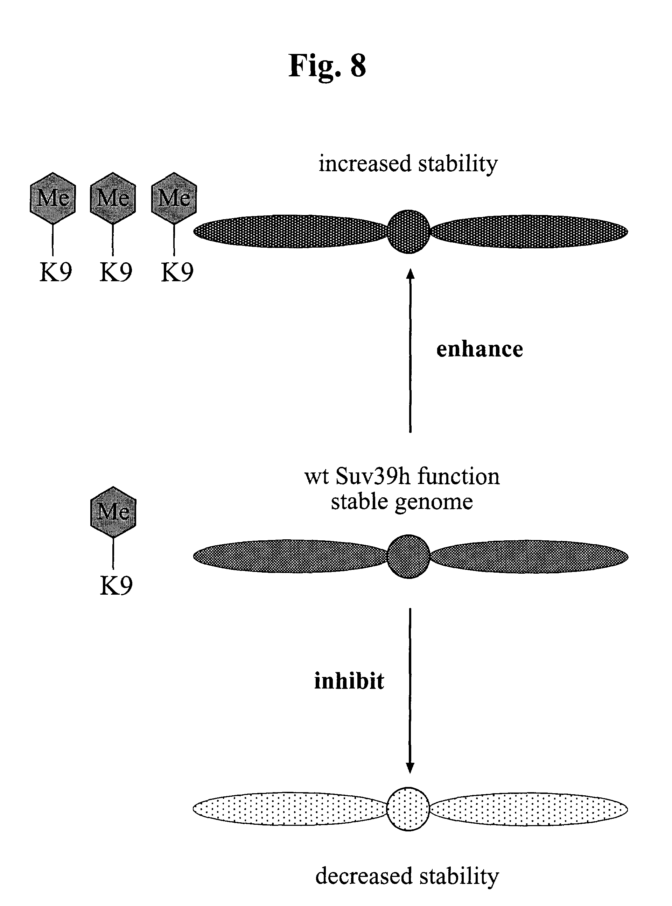

Compounds inhibiting Suv39h HMTase activity result in decreased genome stability and can be used in therapy, optionally in combination with other genome destabilising agents, to target highly proliferative tumour cells. Likewise, agents which enhance Suv39h HMTase activity can be used to stabilise the genome of inherently unstable cells rendering them less prone to acquiring proliferation promoting mutations. A model for *Suv39h* function and effects of inhibition or enhancement of Suv39h enzymes is shown in Figure 8.

Compounds inhibiting Suv39h HMTase activity result in decreased genome stability and can be used in therapy, optionally in combination with other genome destabilising agents, to target highly proliferative tumour cells. Likewise, agents which enhance Suv39h HMTase activity can be used to stabilise the genome of inherently unstable cells, rendering them less prone to acquiring proliferation promoting mutations.

The efficacy of compounds identified as Suv39h modulators can be tested for *in vivo* efficacy in mammalian cells with *Suv39h* double null cells serving as a positive control. Effective compounds should interfere with chromosome stability and segregation, which can be measured by karyotyping, e.g. by analysing the DNA content by FACS or standard cytological techniques. Substances whose potential for therapeutic use has been confirmed in such secondary screens can be further tested for their effect on tumour cells. To test the inhibition of tumour cell proliferation, primary human tumour cells are incubated with the compound identified in the screen and the inhibition of tumour cell proliferation is tested by conventional methods, e.g. bromo-desoxy-uridine or ³H thymidine incorporation. Compounds that exhibit an anti-proliferative effect in these assays may be further tested in tumour animal models and used for the therapy of tumours.

Toxicity and therapeutic efficacy of the compounds identified as drug candidates by the method of the invention can be determined by standard pharmaceutical procedures, which include conducting cell culture and animal experiments to determine the IC₅₀, LD₅0, the ED₅₀. The data obtained are used for determining the human dose range, which will also depend on the dosage form (tablets, capsules, aerosol sprays, ampules, etc.) and the administration route (oral, buccal, nasal, paterental or rectal). A pharmaceutical composition containing the compound as the active ingredient can be formulated in conventional manner using one or more physologically active carriers and excipients. Methods for making such formulations can be found in manuals, e.g. "Remington Pharmaceutical Sciences".

As *Suv39h* is required to maintain a stable karyotype, it can be considered as a tumour suppressor gene. If *Suv39h* mutations prove to be a factor underlying cellular transformation events - which can be confirmed by analysis of *Suv39h* double deficient mice for tumour development and progression -, it can be expected that the re-introduction of a wild type *Suv39h* gene by gene therapy results in increased genomic stability delaying or inhibiting cancer progression.

For gene therapy, the Suv39h DNA molecule may be administered, preferably contained on a plasmid in recombinant form, directly or as part of a recombinant virus or bacterium. In principle, any method of gene therapy may be used for applying Suv39h recombinant DNA, both *in vivo* and *ex vivo.*

Examples of *in vivo* administration are the direct injection of "naked" DNA, either by intramuscular route or using gene guns. Examples of recombinant organisms are vaccinia virus or adenovirus. Moreover, synthetic carriers for nucleic acids such as cationic lipids, microspheres, micropellets or liposomes may be used for *in vivo* administration of nucleic acid molecules coding for the Suv39h polypeptide.

Since it has been shown in the present invention that Suv39h mediates dynamic transitions in higher-order mammalian chromatin in part through its intrinsic HMTase activity, K9 methylation of histone H3 (K9-Me) represents an important epigenetic imprint for chromosome dynamics during cell division. Hence, antibodies specific for K9-Me can be used to screen cells/patients for heterochromatin based genome instabilities. In essence, K9-methylation specific anti-sera can be used as a diagnostic tool for several potential human diseases.

### Brief description of figures:

- Figure 1:: HMTase activity of transfected and recombinant SUV39H1 and Suv39h1 proteins.
- Figure 2:: Specific HMTase activity of the SET domain of mammalian SU(VAR)3-9 related proteins.
- Figure 3:: Lysine 9 of the H3 N-terminus is the major site for *in vitro* methylation by recombinant Suv39h1.
- Figure 4:: Targeting *Suv39h1* and *Suv39h2* in the mouse germline.
- Figure 5:: Analysis of *Suv39h* double null PMEFs.
- Figure 6:: Genomic instabilities in *Suv39h* double null PMEFs.
- Figure 7:: Increased phosH3 phosphorylation in *Suv39h* double null PMEFs.
- Figure 8:: Model for Suv39h function.

### Materials and Methods

a) Sequence alignments and secondary structure predictions
   The SET domains of human SUV39H1 (Aagaard *et al*., 1999), *Drosophila* SU(VAR)3-9 (Tschiersch *et al*., 1994) and *S.pombe* CLR4 (Ivanova *et al*., 1998) were used as a multiple starting alignment for database similarity searches using Profile (Birney *et al*., 1996), hidden Markov (Eddy, 1998) and position-specific iterative BLAST (Altschul *et al*., 1997) methods (representative listings are available from the SET domain page of the SMART WWW-server (Schultz *et al*., 2000). These searches revealed significant similarities to six plant proteins (accession numbers Q43088, 065218, P94026, 080013, AAC29137 and AC007576_12) described as putative lysine N-methyltransferases. For example, a PSI-BLAST search with the *S.pombe* hypothetical protein SPAC3c7.09 as query identified these plant sequences and well-known SET domain sequences within ten rounds using an E-value inclusion threshold of 0.001. The same search also revealed the presence of a SET domain in YHR109w (which is known to encode a cytochrome c MTase (Martzen *et al*., 1999)) within three rounds. Consensus secondary structures were predicted by described algorithms (Frishman and Argos, 1997).
b) Epitope-tagged SUV39H1 proteins in HeLa cells
   The HeLa cell lines overexpressing full-length (myc)3-SUV39H1 (aa 3-412) or (myc)3-Nchromo (aa 3-118) have been described (Aagaard *et al*., 1999; Melcher *et al*., 2000). Nuclear extracts were immunoprecipitated with anti-myc antibody beads (Aagaard *et al*., 1999), and approximately 1-3 µg of matrix-bound (myc)3-tagged SUV39H1 proteins were used for *in vitro* HMTase assays.
c) Generation and purification of GST-fusion proteins
   The GST-Suv1(82-412) product expressed from the pGEX-2T vector (Pharmacia) as a glutathione-S-transferase (GST) fusion protein has been described (Aagaard *et al*., 1999). Additional GST constructs were generated by transferring BamHI-EcoRI PCR amplicons into pGEX-2T, encoding in-frame fusions for Suv39h1(7-221), SUV39H1(82-412), SUV39H1(82-378) ΔC-tail, SUV39H1(255-412) Δcys, Suv39h2(157-477) (O'Carroll *et al*., submitted), CLR4(127-490) (Ivanova *et al*., 1998), EZH2(382-747) (Laible *et al*., 1997) and HRX(3643-3969) (Tkachuk et *al*., 1992). Short internal deletions (ΔNHSCDPN₃₂₃₋₃₂₉ and
   ΔGEELTFDY₃₅₈₋₃₆₅) or point mutations within the -₃₂₀HφφNHSC₃₂₆-motif were directly engineered in the GST-SUV39H1(82-412) plasmid by double PCR mutagenesis. All constructs were confirmed by sequencing.
   Recombinant proteins were expressed in 1I cultures of *E.coli* strain BL21 and solubilized in 10 ml RIPA buffer [(20 mM Tris pH 7.5, 500 mM NaCI, 5 mM EDTA, 1% NP-40, 0.5% sodium deoxycholate) containing a full set of protease inhibitors (Boehringer Mannheim) and lysozyme (5 mg/ml; Sigma)] by freeze-thawing in liquid N², followed by sonication. Soluble proteins were cleared by centrifugation, purified with 800 µl glutathione Sepharose beads (Pharmacia) and washed twice in RIPA buffer. Protein concentration was determined by Coomassie staining of SDS-PAGE gels. Matrix-bound fusion proteins were used immediately for *in vitro* HMTase assays or stored at 4°C.
d) *In vitro* histone methyltransferase (HMTase) assay
   *In vitro* HMTase reactions were modified based on described protocols (Strahl *et al*., 1999) and carried out in a volume of 50 µl of methylase activity buffer (MAB: 50 mM Tris pH 8.5, 20 mM KCI, 10 mM MgCl₂, 10 mM β-ME, 250 mM sucrose), containing 10 µg of free histones (mixture of H1, H3, H2B, H2A and H4; Boehringer Mannheim) as substrates and 300 nCi S-adenosyl-[*methyl*-¹⁴C]-L-methionine (25 µCi/ml) (Amersham) as methyl donor. 10 µg of matrix-bound GST-fusion proteins were routinely used to assay for HMTase activity. After incubation for 60 min. at 37°C, reactions were stopped by boiling in SDS loading buffer, and proteins were separated by 15% or 18% SDS-PAGE and visualised by Coomassie staining and fluorography.
   HMTase assays with individual histones (Boehringer Mannheim), insulin (Sigma) or N-terminal peptides were performed with 5 µg of substrate. The following peptides were used: wild-type N-terminus of human histone H3 (ARTKQTARKSTG-GKAPRKQL) (SEQ ID NO:7) and mutant peptide which changes lysine 9 (bold) to leucine; N-terminus of human CENP-A (MGPRRRSRKPEAPRRRSPSP) (SEQ ID NO:8) (Sullivan *et al*., 1994); N-terminus of rat macro-H2A (MSSRGGKKKSTKTSRSAKAG) (SEQ ID NO:9) (Pehrson and Fried, 1992).
   Peptide microsequencing of the *in vitro* methylated wild-type H3 N-terminal peptide and determination of ³H-incorporation of individual amino acids by scintillation counting was done as described (Strahl *et al*., 1999).
e) Generation of Suv39h1 and Suv39h2 deficient mice by gene targeting
   *Suv39h1* maps to the X-chromosome. The cloned partial *Suv39h1* genomic locus (O'Carroll *et al*., submitted) was used to generate a targeting construct. A1.2kb Pfu PCR amplicon, generated with the primers gM3-9(SII) and gM3-9(RI), was used as a short arm of homology and cloned in frame with the nls-lacZ gene of the pGNA-T vector. This places the first 3 amino acids of exon 2 in frame with the nls-lacZ gene generating a fusion protein of the first 8 amino acids of Suv39h1 and lacZ from the targeted locus. A 5.4kb Sacl (filled in) fragment from *Suv39h1* genomic subclone *gSuv39h1* #18 was used as a long arm of homology.
   *Suv39h2 is* autosomal and maps to chromosome 2. The cloned partial *Suv39h2* genomic locus (O'Carroll *et al.,* submitted) was used to generate a targeting construct. A1.4 kb Pfu PCR amplicon, generated with the primers Suv2SII and Suv2RI, was used as a short arm of homology and cloned in frame with the nls-lacZ gene of the pGNA-T vector. This places the first 113 amino acids of exon 2 in frame with the nls-lacZ gene generating a fusion protein of the first 113 amino acids of Suv39h2 and lacZ from the targeted locus. A 4.9kb Mlul/Apal (filled in) fragment from *Suv39h2* genomic subclone *gSuv39h2* #28 was used as long arm of homology to inactivate the locus.
   These constructs were linearised with NotI, electroporated into R1 ES cells (*Suv39h1*) and E14.1 ES cells *(Suv39h2),* ES cells were put under G418 selection and G418 resistant colonies screened for homologous recombination by PCR and Southern blot analysis. Targeted feeder dependent ES cell clones were injected into blastocysts of C57BL/6 mice and reimplanted into pseudopregnant females to produce chimeric offspring. Germ-line transmission was obtained after a backcross between chimeric males and C57BL/6 females. Heterozygous mice were interbred to obtain *Suv39h1* and *Suv39h2* deficient mice. *Suv39h1* and *Suv39h2* deficient mice were then interbred to generate *Suv39h* double deficient mice.
f) Generation and analysis of *Suv39h* double null primary mouse embryonic fibroblasts (PMEFs)
   PMEFs were derived from day E12.5 *Suv39h* double null embryos obtained after intercrossing *Suv39h1*^{*-/-*}*ISuv39h2*^{*+/-*} compound mutant mice. As controls, PMEFs were prepared from wild-type embryos of the same genetic background. For cell cycle profiles and growth curve analysis, passage 2 PMEFs were analyzed as described (Xu *et al*., 1999). Staining of PMEF interphase chromatin with anti-phosH3 (Hendzel *et al*., 1997) antibodies was done in untreated cells. For the biochemical analysis, total nuclear extracts were precalibrated by Ponceau staining, immuno-blotted with anti-H3 (Upstate Biotechnology) and anti-phosH3 (Hendzel *et al*., 1997) antibodies and visualised by peroxidase staining using Enhanced ChemiLuminescence (ECL) (Amersham).

### Example 1

### Sequence similarity of SET domains with plant methyltransferases

Using the SET domains of the SU(VAR)3-9 protein family as a starting alignment, significant sequence and secondary structure similarities (see Methods) to six plant protein methyltransferases were detected. Although some of these plant sequences have been classified as potential histone lysine N-methyltransferases, only one had been functionally characterised but was found to lack HMTase activity (Klein and Houtz, 1995; Zheng *et al*., 1998).

Detected were amino acid and secondary structure [β-sheet (b) or α-helix (h)] similarities of the C-terminal halves of SET domain sequences from human SUV39H1 (Aagaard *et al*., 1999) (AF019968), murine Suv39h1 (Aagaard *et al*., 1999) (AF019969), murine Suv39h2 (O'Carroll *et al*., submitted), (AF149205), *Drosophila* SU(VAR)3-9 (Tschiersch *et al*., 1994) (P45975), a *C.elegans* SU(VAR)3-9-like ORF C15H11.5 (CAB02737), *S.pombe* CLR4 (Ivanova *et al.,* 1998) (074565), human EZH2 (Laible *et al*., 1997) (Q15910), the human trithorax homologue HRX (Tkachuk *et al*., 1992) (Q03164), and MTases from *P.sativum* (rubisco Is-MT; Q43088) (Klein and Houtz, 1995; Zheng *et al*., 1998) and *A.thaliana* (065218). The plant MTase sequences contain an insertion of approximately 100 amino acids in the middle of the SET domain.

### Example 2

HMTase activity of transfected and recombinant SUV39H1 and Suv39h1 proteins.

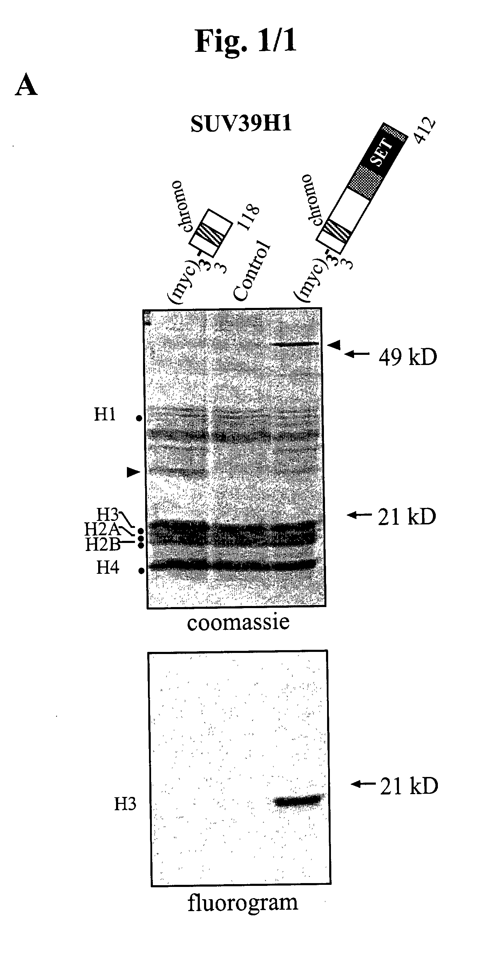

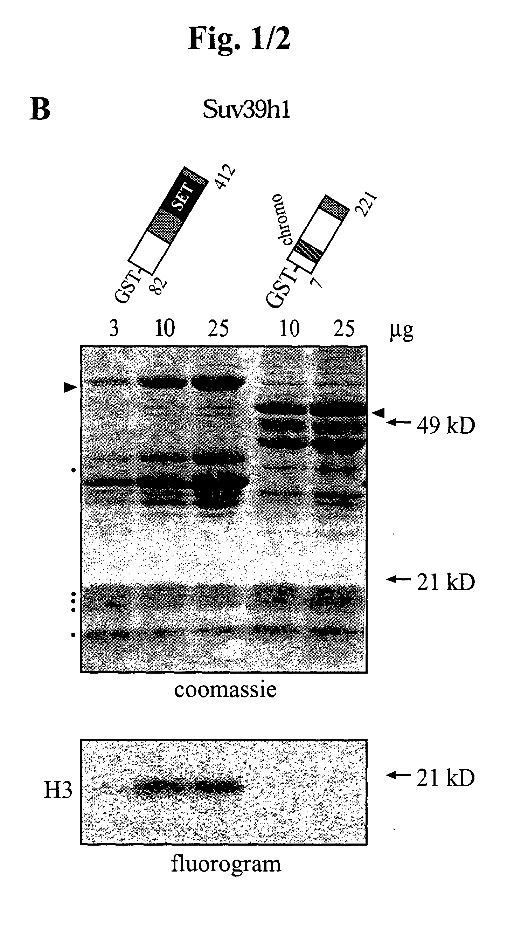

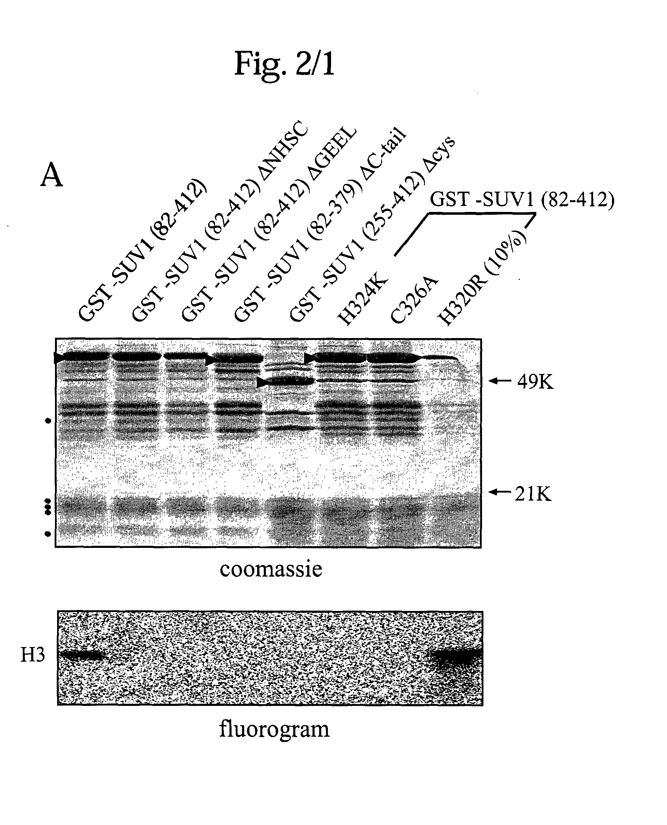

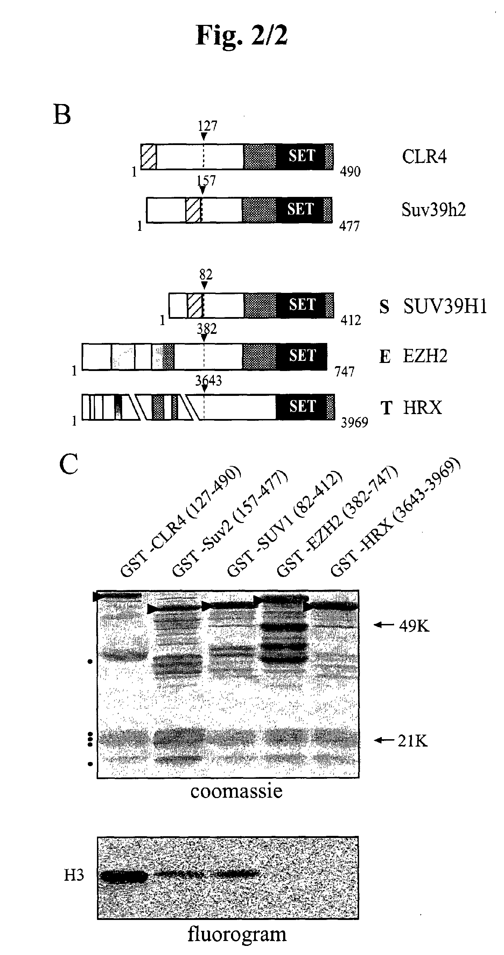

To investigate whether the SET domain of human SUV39H1 has enzymatic activity, histones were tested as possible substrates for *in vitro* methylation. Using HeLa cell lines 'stably' expressing triple myc-tagged full-length SUV39H1 (aa 3-412), the ectopic protein was enriched from nuclear extracts by immunoprecipitation with anti-myc beads (see Figure 1/1A, arrowhead top panel) and probed for activity to transfer a labelled methyl group from S-adenosyl-[*methyl*-¹⁴C]-L-methionine to free histones according to described conditions (Strahl *et al*., 1999). Reaction products were separated by SDS-PAGE and visualised by fluorography, indicating selective transfer of the methyl-label to H3 (Figure 1/1A, lower panel). By contrast, no signals were detected with extracts from a HeLa cell line that expresses only the N-terminal third of SUV39H1 (aa 3-118) or with extracts from HeLa control cells. To confirm that the HMTase activity is an intrinsic property of SUV39H1 and not mediated by a SUV39H1-associated factor, the *in vitro* HMTase reactions was repeated with recombinant products that were purified as GST-fusion proteins from *E.coli* (see Figure 1/2B, arrowheads top panel). For this analysis, murine Suv39h1, which is 95% identical to human SUV39H1 (Aagaard *et al*., 1999) was used. A purified GST-product comprising aa 82-412 maintained HMTase activity (although at a reduced level as compared to transfected SUV39H1), whereas a purified GST-product comprising aa 7-221 proved negative, even at higher protein concentrations (Figure 1/2B, lower panel). These results suggest that the HMTase activity resides in the C-terminal SET domain.

In Fig. 1A, triple myc-tagged full-length human SUV39H1 (aa 3-412) or a C-terminally truncated SUV39H1 protein (aa 3-118) were immunoprecipitated from 'stably' transfected HeLa cell lines with anti-myc antibody beads and used in *in vitro* HMTase reactions with free histones as substrates and S-adenosyl-[*methyl*-¹⁴C]-L-methionine as methyl donor. The Coomassie stain (top panel) shows purified proteins by arrowheads and free histones by dots. Fluorography (bottom panel, Figure 1A) indicates HMTase activity of (myc)3-SUV1 (3-412).

In the experiments shown in Fig. 1B, recombinant GST-fusion proteins encoding different domains of murine Suv39h1were used in increasing protein concentrations for *in vitro* HMTase reactions as described above.

### Example 3

Definition of a catalytic motif in the SET domain of human SUV39H1.

Similar to the recombinant murine GST-Suv39h1(82-412) product, the corresponding human SUV39H1 fusion protein [GST-SUV39H1(82-412)] is catalytically active (see Figure 2). Short internal deletions (ΔNHSCDPN₃₂₃₋ ₃₂₉; ΔNHSC and ΔGEELTFDY₃₅₈₋₃₆₅; ΔGEEL) were introduced into the two conserved regions of the SET domain core in GST-SUV39H11(82-412) and, in addition, mutants that lack the C-terminal tail (DC-tail) or the SET-associated cysteine-rich region (□cys) were also generated. All mutant proteins failed to demonstrate HMTase activity (see Figure 2/1A). To investigate enzyme function of the SET domain in more detail, point mutations were introduced into the most highly conserved motif. *In vitro* HMTase assays indicated that all point mutations, with the exception of one, abolished enzymatic activity. Surprisingly, the latter mutation (H320R) resulted in an hyperactive enzyme with approximately 20-fold increased activity. The data obtained define the ₃₂₀H□□NHSC₃₂₆ motif in the SET domain as an important catalytic site.

Specific HMTase activity of the SET domain of mammalian SU(VAR)3-9 related proteins.

Because the SET domain is one of the most conserved protein motifs in chromatin regulators (Stassen *et al*., 1995; Jenuwein *et al*., 1998), it was next analyzed whether SU(VAR)3-9 family members or other SET domain proteins contain HMTase activity. GST-fusion products of the extended SET domains of *S.pombe* CLR4 (Ivanova *et al.,* 1998), human EZH2 (Laible *et al*., 1997) and human HRX (Tkachuk *et al*., 1992) were generated that would correspond to GST-SUV39H1(82-412) (see Figure 2/2B). Interestingly, GST-CLR4(127-490) displayed pronounced HMTase activity at three- to five-fold increased levels (see Figure 2/2C) as compared to the recombinant SUV39H1 product, consistent with CLR4 carrying an arginine at the hyperactive position. By contrast, both GST-EZH2(382-747) and GST-HRX(3643-3966) had undetectable HMTase activity towards free histones (Figure 2/2C), whereas a comparable GST product generated from the recently isolated murine *Suv39h2* gene (O'Carroll et al., submitted), GST-Suv39h2(157-477), was as active as GST-SUV39H1(82-412). EZH2 lacks the C-terminal cysteines, and HRX does not contain the SET associated cysteine-rich region (Figure 2B). Both of these cysteine domains are present in CLR4, Suv39h2 and SUV39H1. In agreement with the mutational analysis of SUV39H1, it thus appears that HMTase activity towards free histones requires the combination of the SET domain with adjacent cysteine-rich regions, and is a quality found in only a restricted number of SET domain containing proteins.

In Fig. 2A, approximately 10 µg of the indicated fusion proteins encoding GST-SUV1(82-412) (= human SUV39H1) and seven SET domain mutants were used in *in vitro* HMTase reactions with free histones as outlined in Figure 1. For the hyperactive H320R mutant, only 1 µg (10%) of the corresponding fusion product was used. Fig 2 shows a diagram representing the domain structures of CLR4, Suv39h2, SUV39H1, EZH2 and HRX proteins, with the arrowheads demarcating the N-terminal fusion to GST. Cysteine-rich regions are indicated by grey stippling.

In Fig. 2C, approximately 10 µg of the indicated fusion proteins encoding *S.pombe* CLR4 [GST-CLR4(127-490)], murine Suv39h2 [GST-Suv2(157-477)], human EZH2 [GST-EZH2(382-747)], human HRX [GST-HRX(3643-3969)] and human SUV39H1 [GST-SUV1 (82-402)] were used in *in vitro* HMTase reactions with free histones as outlined in Figure 1.

### Example 4

Lysine 9 of the H3 N-terminus is the major site for *in vitro* methylation by recombinant Suv39h1.

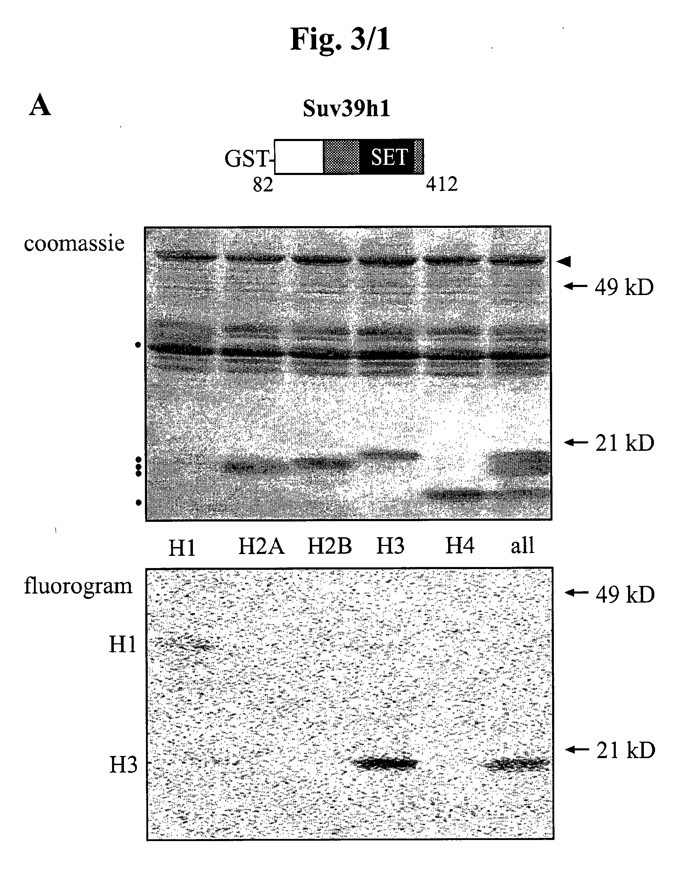

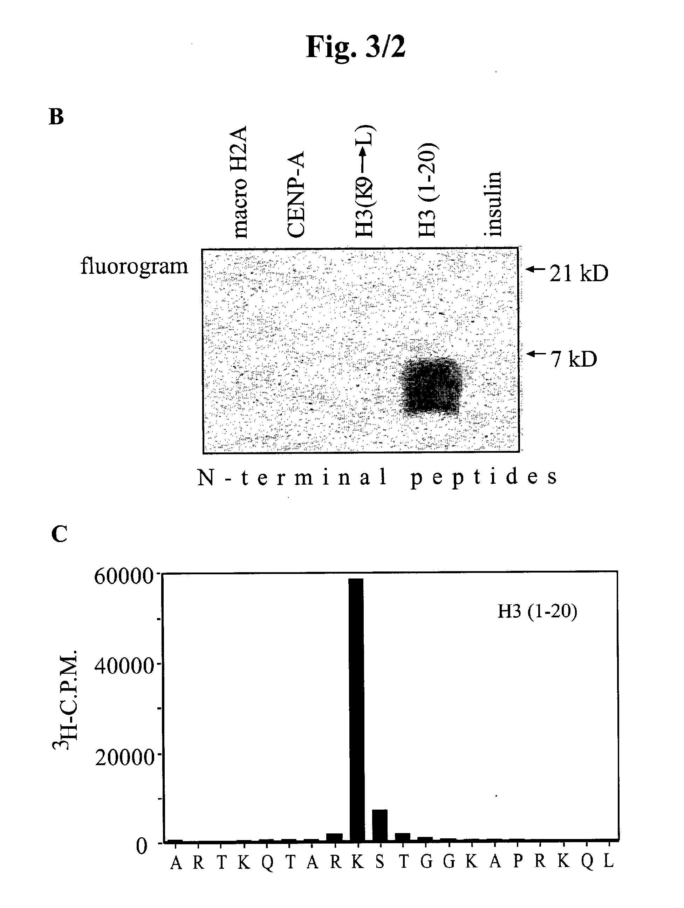

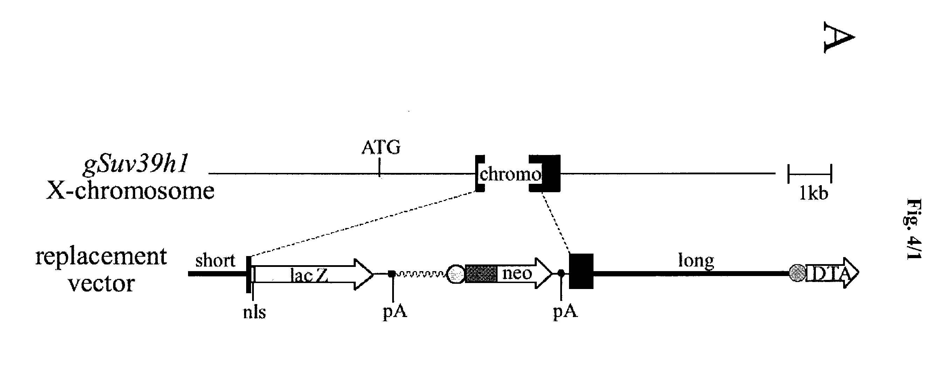

The above Examples indicated that the HMTase activity of mammalian SU(VAR)3-9 related proteins is selective for H3 under the chosen assay conditions. To examine this finding in more detail, *in vitro* methylation reactions were performed with individual histones, using GST-Suv39h1(82-412) as an enzyme. As shown in Figure 3/1A, H3 is specifically methylated by GST-Suv39h1(82-412), whereas no signals are detected with H2A, H2B or H4. A weak signal is present if H1 was used as the sole substrate; the significance of H1 methylation remains to be determined. Methylation of H3 has been shown to occur predominantly at lysine 4 in a wide range of organisms, as well as at lysine 9 in HeLa cells, although the responsible HMTase(s) have yet to be defined (Strahl *et al*., 1999). To investigate the site utilisation profile of Suv39h1, unmodified peptides comprising the wild-type H3 N-terminus (aa 1-20) and a mutant K9L peptide, changing lysine 9 to leucine were tested as substrates. Additionally, insulin and peptides comprising the N-termini of CENP-A (Sullivan *et al*., 1994) and macroH2A (Pehrson and Fried, 1992) were included. Peptides were *in vitro* methylated by GST-Suv39h1(82-412), and reaction products were separated by high percentage SDS-PAGE and visualised by fluorography. These *in vitro* assays revealed selective methylation of the wild-type H3 peptide, whereas no signals were detected with the CENP-A or macroH2A peptides, or with insulin (see Figure 3/2B). Importantly, the mutated H3 (K9L) peptide was not a substrate, suggesting that lysine 9 of the H3 N-terminus is a preferred residue for Suv39h1-dependent HMTase activity.

To more definitively determine this site preference, the wild-type H3 N-terminal peptide was *in vitro* methylated by GST-Suv39h1(82-412), using S-adenosyl-[*methyl*-³H]-L-methionine. The labelled peptide, purified by reverse-phase HPLC, was then directly microsequenced, and
³H-incorporation associated with each individual amino acid was analysed by scintillation counting. The results confirmed selective transfer of methyl-label to lysine 9 (see Figure 3/2C), demonstrating that Suv39h1 is a highly site-specific HMTase for the H3 N-terminus *in vitro.*

In Fig. 3A, approximately 10 µg of murine GST-Suv39h1(82-412) were used in *in vitro* HMTase reactions with individual histones as outlined in Figure 1.

Fig 3B shows the results of *in vitro* methylation assays using GST-Suv39h1(82-412) as enzyme and the indicated N-terminal peptides of wild-type H3, mutated H3 (K9L), CENP-A, macroH2A or insulin as substrates.

Fig 3C shows the result of automated sequencing of the wild-type H3 N-terminal peptide (aa 1-20) that had been methylated *in vitro* by recombinant GST-Suv39h1(82-412). Displayed is the ³H-incorporation of individual amino acids identified at each successive round of microsequencing.

### Example 5

Targeting the *Suv39h1* and *Suv39h2* loci in the mouse germline.

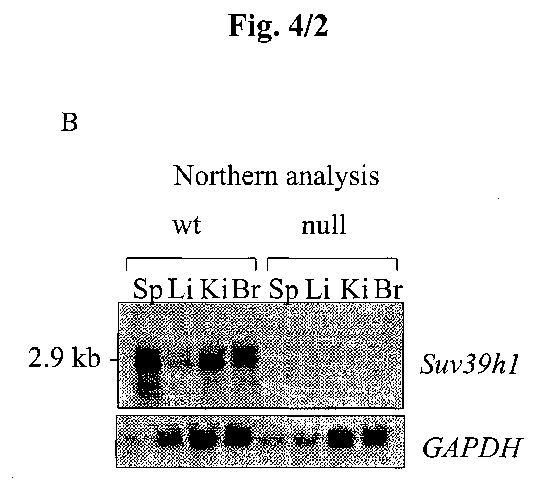

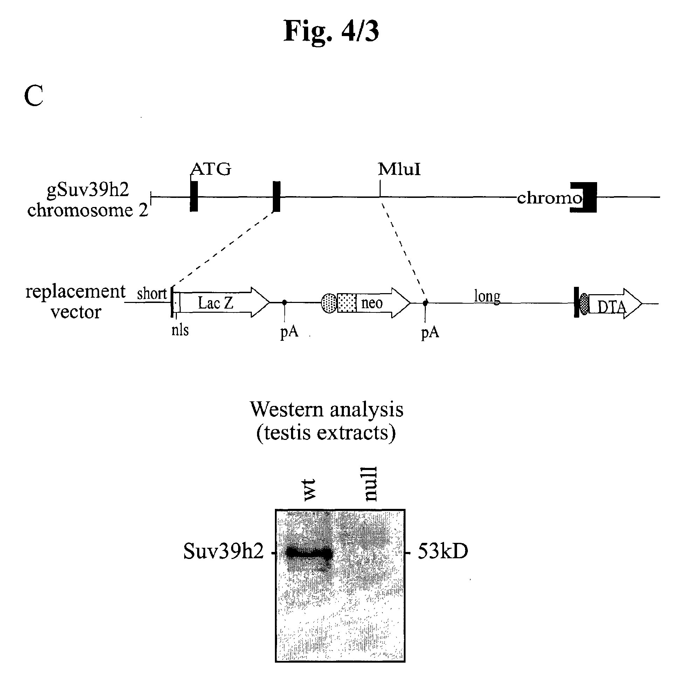

Murine *Suv39h* genes are encoded by 2 loci, *Suv39h1* and *Suv39h2* (O'Carroll *et al*., submitted).To investigate the *in vivo* significance of *Suv39h* function and *Suv39h* dependent K9 H3 methylation, mouse strains deficient for both *Suv39h1* and *Suv39h2* were generated according to standard techniques. The targeting strategies are shown in Figure 4, as well as demonstrating the production of null alleles for both *Suv39h1* and *Suv39h2.* Mutation of either gene results in viable and fertile mice as a consequence of functional redundancy between both loci. Therefore, *Suv39h1* and *Suv39h2* deficient strains were intercrossed to produce *Suv39h* double deficient mice.

Double mutant mice are born in sub-Mendelian ratios, approximately 20% of the expected double mutants are observed. Some double null embryos exhibit severe growth retardation and exencephaly. In addition, surviving double mutants are growth retarded, suggesting a role for *Suv39h* in cell proliferation.

Fig. 4 shows a conventional targeting strategy used to inactivate the X-linked *Suv39h1* locus. Fig. 4B shows the Northern blot analysis of *Suv39h1* from spleen (Sp), liver (Li), kidney (Kidney), and brain (Br) from wild-type and *Suv39h1* null mice. Fig. 4C shows the conventional targeting strategy used to inactivate the autosomal *Suv39h2* locus.(Bottom panel) Western blot analysis with anti-Suv39h2 antibodies on protein extracts derived from wild-type and *Suv39h2* null testis.

### Example 6

Increased aneuploidies in Suv39h double null primary mouse fibroblasts (PMEFs).

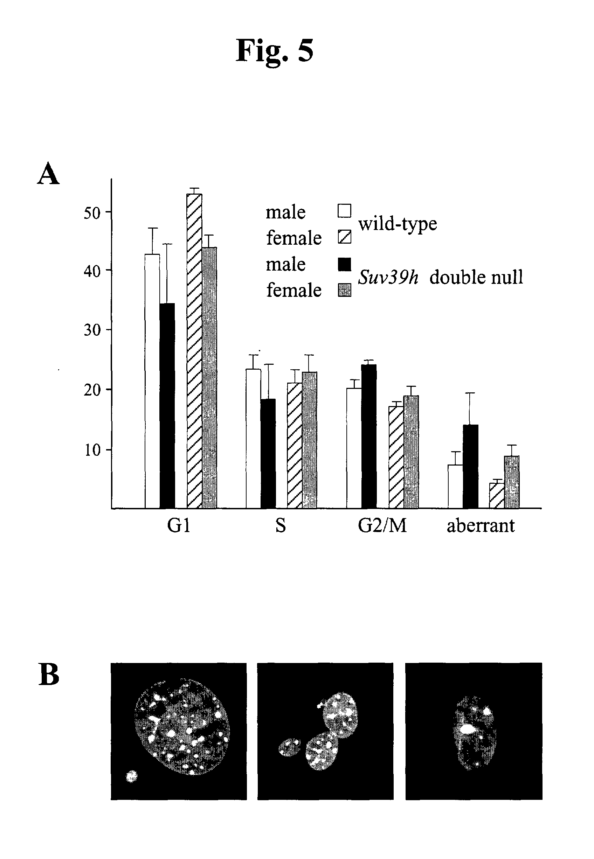

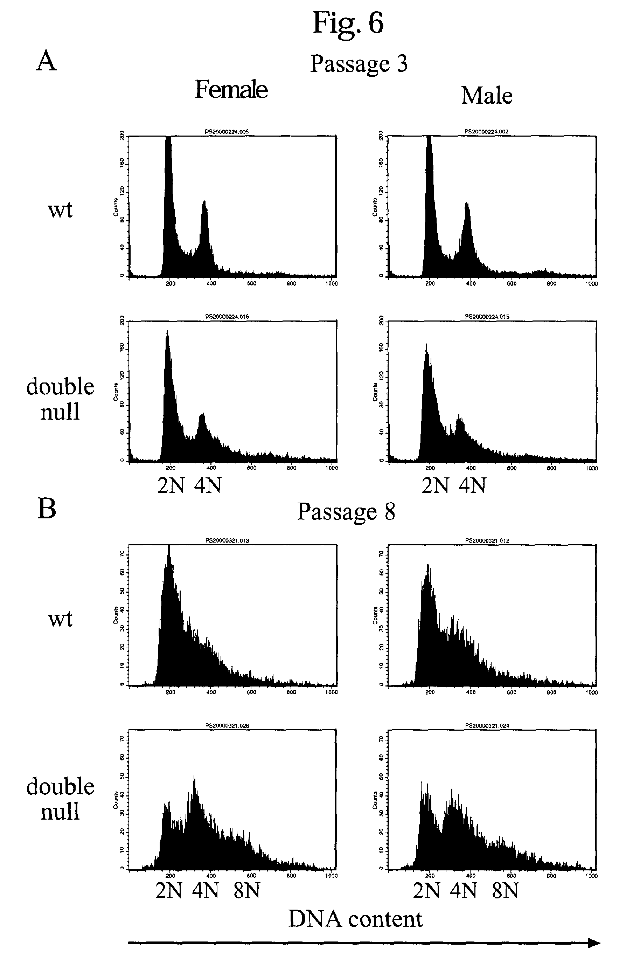

In order to determine whether the embryonic phenotypes in *Suv39h* double null mice can be attributed to mitotic defects, PMEFs derived from *Suv39h* double mice were analysed. Cell cycle profiles of wild-type and *Suv39h* double null PMEFs indicated rather similar percentages of cells to be in S- and G2/M-phases (see Figure 5A), whereas *Suv39h* double null PMEFs display a reduced G1-index and an increased proportion of cells with aberrant nuclear morphologies, reminiscent of division defects during mitosis. For example, *Suv39h* double null PMEFs contain approximately two-fold elevated numbers of cells with micro- and polynuclei, and are further characterised by cell subpopulations with oversized nuclei or a weak definition of heterochromatin that appears in only a few unusually condensed foci (see Figure 5B). Furthermore, double null cells also show genomic instabilities and readily become aneuploid. The severity of these aneuploidies increases with higher passage numbers (see Figure 6). The inability of double null cells to maintain a stable karyotype may underlie the *Suv39h* embryonic phenotype.

Fig. 5A shows the percentages of cells in various cell cycle stages of wild-type and *Suv39h* double null PMEFs.

Fig. 5B shows representative images (left and middle) of aberrant mitoses in *Suv39h* double null PMEFs detected by β-tubulin (not shown) and DAPI staining. Also shown (right image) is a nucleus exemplifying the unusual definition of heterochromatin in a subpopulation of *Suv39h* double null PMEFs. All images were taken at a magnification of 630x.

Fig. 6A shows the DNA content profile of wild-type and *Suv39h* double null PMEFs at passage 3. Fig. 6B shows the DNA content profile of wild-type and *Suv39h* double null PMEFs at passage 8.

### Example 7

Increased phosH3 phosphorylation in *Suv39h* double null PMEFs.

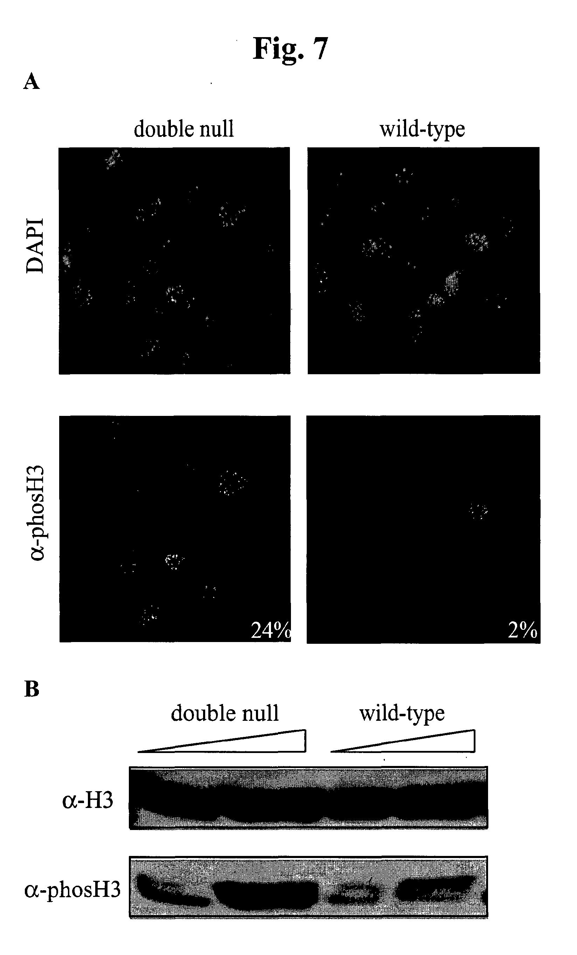

Phosphorylation at serine 10 in the N-terminal tail of H3 (phosH3) has been shown to be required for condensation and subsequent segregation of chromosomes (Wei *et al*., 1999). During the cell cycle, phosH3 initiates within pericentric heterochromatin in late G2 and then progresses along the entire chromosomes during mitosis (Hendzel *et al*., 1997). In wild-type PMEFs, approximately 7% of the cells stain positive for the characteristic, heterochromatin-associated phosH3 foci, as detected by indirect immunofluorescence with anti-phosH3 antibodies (see Figure 7A, right panel). In contrast, this number is increased by a factor of about 3-fold in *Suv39h* double null PMEFs, with approximately 22% of the cells containing phosH3-positive foci (Figure 7A, left panel), although their definition appears in many small speckles which do not always overlap with DAPI-dense material (data not shown). This result suggested that the overall levels of phosH3 may be enhanced in *Suv39h* double null PMEFs. Therefore, the relative abundance of phosH3 in precalibrated nuclear extracts was determined with anti-phosH3-specific antibodies. This quantitation indicated a significantly higher level of phosH3 in *Suv39h* double null cells as compared to wild-type controls (see Figure 7B). Together, the obtained data are most consistent with a model in which Suv39h-mediated methylation of lysine 9 in H3 negatively regulates phosphorylation of serine 10.

Fig. 7A shows an interphase chromatin staining with anti-phosH3 antibodies and CY3-conjugated secondary antibodies. DNA was counterstained with DAPI. At least 1,000 cells were counted to evaluate the percentage (indicated in the Figure) of anti-phosH3 positive cells. Fig 7B shows a quantitative Western analysis with 15 µg and 30 µg of total nuclear proteins immuno-blotted with anti-H3 and anti-phosH3 antibodies.

### REFERENCES

Aagaard, L., Laible, G., Selenko, P., Schmid, M., Dorn, R., Schotta, G., Kuhfittig, S., Wolf, A., Lebersorger, A., Singh, P.B., Reuter, G. and Jenuwein, T. (1999) Functional mammalian homologues of the Drosophila PEV-modifier Su(var)3- 9 encode centromere-associated proteins which complex with the heterochromatin component M31. *Embo J*, **18**, 1923-38.
Aasland, R. and Stewart, A.F. (1995) The chromo shadow domain, a second chromo domain in heterochromatin- binding protein 1, HP1. *Nucleic Acids Res,* **23,** 3168-74.
Allshire, R.C., Nimmo, E.R., Ekwall, K., Javerzat, J.P. and Cranston, G. (1995) Mutations derepressing silent centromeric domains in fission yeast disrupt chromosome segregation. *Genes Dev*, **9,** 218-33.
Altschul, S.F., Madden, T.L., Schaffer, A.A., Zhang, J., Zhang, Z., Miller, W. and Lipman, D.J. (1997) Gapped BLAST and PSI-BLAST: a new generation of protein database search programs. *Nucleic Acids Res,* **25,** 3389-402.
Baksa, K., Morawietz, H., Dombradi, V., Axton, M., Taubert, H., Szabo, G., Torok, I., Udvardy, A., Gyurkovics, H., Szoor, B. and et al. (1993) Mutations in the protein phosphatase 1 gene at 87B can differentially affect suppression of position-effect variegation and mitosis in Drosophila melanogaster. *Genetics,* **135,** 117-25.
Ball, L.J., Murzina, N.V., Broadhurst, R.W., Raine, A.R.C., Archer, S.J., Stott, F.J., Murzin, A.G., Singh, P.B., Domaille, P.J. and Laue, E.D. (1997) Structure of the chromatin binding (chromo) domain from mouse modifier protein 1. *EMBO J*, **16,** 2473-2481.
Birney, E., Thompson, J.D. and Gibson, T.J. (1996) PairWise and SearchWise: finding the optimal alignment in a simultaneous comparison of a protein profile against all DNA translation frames. *Nucleic Acids Res,* **24,** 2730-9.
Chen, D., Ma, H., Hong, H., Koh, S.S., Huang, S.M., Schurter, B.T., Aswad, D.W. and Stallcup, M.R. (1999) Regulation of transcription by a protein methyltransferase. *Science,* **284,** 2174-7.
Cléard, F., Delattre, M. and Spierer, P. (1997) SU(VAR)3-7, a Drosophila heterochromatin-associated protein and companion of HP1 in the genomic silencing of position-effect variegation. *Embo J,* **16,** 5280-8.
De Rubertis, F., Kadosh, D., Henchoz, S., Pauli, D., Reuter, G., Struhl, K. and Spierer, P. (1996) The histone deacetylase RPD3 counteracts genomic silencing in Drosophila and yeast. *Nature,* **384,** 589-91.
Eddy, S.R. (1998) Profile hidden Markov models. *Bioinformatics*, **14,** 755-63.
Eissenberg, J.C., Morris, G.D., Reuter, G. and Hartnett, T. (1992) The heterochromatin-associated protein HP-1 is an essential protein in Drosophila with dosage-dependent effects on position-effect variegation. *Genetics,* **131,** 345-52.
Ekwall, K., Nimmo, E.R., Javerzat, J.P., Borgstrom, B., Egel, R., Cranston, G. and Allshire, R. (1996) Mutations in the fission yeast silencing factors clr4+ and rik1+ disrupt the localisation of the chromo domain protein Swi6p and impair centromere function. *J Cell Sci,* **109,** 2637-48.
Frishman, D. and Argos, P. (1997) Seventy-five percent accuracy in protein secondary structure prediction. *Proteins,* **27,** 329-35.
Grunstein, M. (1998) Yeast heterochromatin: regulation of its assembly and inheritance by histones. *Cell,* **93,** 325-8.
Hendzel, M.J., Wei, Y., Mancini, M.A., Van Hooser, A., Ranalli, T., Brinkley, B.R., Bazett-Jones, D.P. and Allis, C.D. (1997) Mitosis-specific phosphorylation of histone H3 initiates primarily within pericentromeric heterochromatin during G2 and spreads in an ordered fashion coincident with mitotic chromosome condensation. *Chromosoma*, **106,** 348-60.
Henikoff, S. (1997) Position effect variegation in Drosophila: recent progress. *Epigenetic mechanisms of gene regulation.* CSHL press.
Ivanova, A.V., Bonaduce, M.J., Ivanov, S.V. and Klar, A.J. (1998) The chromo and SET domains of the Clr4 protein are essential for silencing in fission yeast. *Nat Genet,* **19**, 192-5.
Jacobson, S. and Pillus, L. (1999) Modifying chromatin and concepts of cancer. *Curr Opin Genet Dev*, **9**, 175-84
Jenuwein, T., Laible, G., Dorn, R. and Reuter, G. (1998) SET domain proteins modulate chromatin domains in eu- and heterochromatin. *Cell Mol Life Sci,* **54,** 80-93.
Karpen, G.H. and Allshire, R.C. (1997) The case for epigenetic effects on centromere identity and function. *TIG*, **13,** 489-496.
Klein, R.R. and Houtz, R.L. (1995) Cloning and developmental expression of pea ribulose-1,5-bisphosphate carboxylase/oxygenase large subunit N-methyltransferase. *Plant Mol Biol,* **27,** 249-61.
Koonin, E.V., Zhou, S. and Lucchesi, J.C. (1995) The chromo superfamily: new members, duplication of the chromo domain and possible role in delivering transcription regulators to chromatin. *Nucleic Acids Res,* **23,** 4229-33.
Laible, G., Wolf, A., Dorn, R., Reuter, G., Nislow, C., Lebersorger, A., Popkin, D., Pillus, L. and Jenuwein, T. (1997) Mammalian homologues of the Polycomb-group gene Enhancer of zeste mediate gene silencing in Drosophila heterochromatin and at S. cerevisiae telomeres. *Embo J,* **16,** 3219-32.
Larsson, J., Zhang, J. and Rasmuson-Lestander, A. (1996) Mutations in the Drosophila melanogaster gene encoding S-adenosylmethionine synthetase [corrected] suppress position-effect variegation [published erratum appears in Genetics 1996 Nov;144(3):1329]. *Genetics,* **143,** 887-96.
Lengauer, C., Kinzler, K.W. and Vogelstein, B. (1997) Genetic instability in colorectal cancers. *Nature,* **386**, 623-7.
Martzen, M.R., McCraith, S.M., Spinelli, S.L., Torres, F.M., Fields, S., Grayhack, E.J. and Phizicky, E.M. (1999) A biochemical genomics approach for identifying genes by the activity of their products. *Science,* **286**, 1153-5.
Melcher, M., Schmid, M., Aagaard, L., Selenko, P., Laible, G. and Jenuwein, T. (2000) Structure-function analysis of SUV39H1 reveals a dominant role in heterochromatin organization, chromosome segregation, and mitotic progression. *Mol Cell Biol,* **20,** 3728-41.
Pehrson, J.R. and Fried, V.A. (1992) MacroH2A, a core histone containing a large nonhistone region. *Science,* **257,** 1398-400.
Platero, J.S., Hartnett, T. and Eissenberg, J.C. (1995) Functional analysis of the chromo domain of HP1. *Embo J,* **14,** 3977-86.
Reuter, G. and Spierer, P. (1992) Position effect variegation and chromatin proteins. *BioEssays*, **14,** 605-612.
Sassone-Corsi, P., Mizzen, C.A., Cheung, P., Crosio, C., Monaco, L., Jacquot, S., Hanauer, A. and Allis, C.D. (1999) Requirement of Rsk-2 for epidermal growth factor-activated phosphorylation of histone H3. *Science,* **285,** 886-91.
Schotta, G. and Reuter, G. (2000) Controlled expression of tagged proteins in Drosophila using a new modular P-element vector system. *Mol Gen Genet,* **262,** 916-20.
Schultz, J., Copley, R.R., Doerks, T., Ponting, C.P. and Bork, P. (2000) SMART: a web-based tool for the study of genetically mobile domains. *Nucleic Acids Res,* **28**, 231-4.
Stassen, M.J., Bailey, D., Nelson, S., Chinwalla, V. and Harte, P.J. (1995) The Drosophila trithorax proteins contain a novel variant of the nuclear receptor type DNA binding domain and an ancient conserved motif found in other chromosomal proteins. *Mech Dev,* **52,** 209-23.
Strahl, B.D. and Allis, C.D. (2000) The language of covalent histone modifications. *Nature,* **403,** 41-5.
Strahl, B.D., Ohba, R., Cook, R.G. and Allis, C.D. (1999) Methylation of histone H3 at lysine 4 is highly conserved and correlates with transcriptionally active nuclei in Tetrahymena. *Proc Natl Acad Sci U S A*, **96,** 14967-72.
Sullivan, K.F., Hechenberger, M. and Masri, K. (1994) Human CENP-A contains a histone H3 related histone fold domain that is required for targeting to the centromere. *J Cell Biol*, **127,** 581-92.
Tkachuk, D.C., Kohler, S. and Cleary, M.L. (1992) Involvement of a homolog of Drosophila trithorax by 11 q23 chromosomal translocations in acute leukemias. *Cell,* **71,** 691-700.
Tschiersch, B., Hofmann, A., Krauss, V., Dorn, R., Korge, G. and Reuter, G. (1994) The protein encoded by the Drosophila position-effect variegation suppressor gene Su(var)3-9 combines domains of antagonistic regulators of homeotic gene complexes. *Embo J,* **13,** 3822-31.
Turner, B.M. (1998) Histone acetylation as an epigenetic determinant of long-term transcriptional competence. *Cell Mol Life Sci,* **54,** 21-31.
Wallrath, L.L. (1998) Unfolding the mysteries of heterochromatin. *Curr Opin Genet Dev,* **8**, 147-53,
Wei, Y., Yu, L., Bowen, J., Gorovsky, M.A. and Allis, C.D. (1999) Phosphorylation of histone H3 is required for proper chromosome condensation and segregation. *Cell*, **97,** 99-109.
Xu, X., Weaver, Z., Linke, S.P., Li, C., Gotay, J., Wang, X.W., Harris, C.C., Ried, T. and Deng, C.X. (1999) Centrosome amplification and a defective G2-M cell cycle checkpoint induce genetic instability in BRCA1 exon 11 isoform-deficient cells. *Mol Cell,* **3**, 389-95.
Zheng, Q., Simel, E.J., Klein, P.E., Royer, M.T. and Houtz, R.L. (1998) Expression, purification, and characterization of recombinant ribulose-1,5-bisphosphate carboxylase/oxygenase large subunit nepsilon-methyltransferase. *Protein Expr Purif*, **14**, 104-12.

## Claims

1. A method for identifying a compound that alters higher order chromatin dependent chromosome stability, said method comprising incubating a substrate for a methyltransferase, in the presence of a methyl donor, with a methyltransferase with Suv39h-like methyltransferase activity, in the presence or absence of a test compound and determining whether the compound modulates the methyltransferase activity.

2. The method of claim 1, wherein the methyltransferase with Suv39h-like activity is murine Suv39h1 or human SUV39H1.

3. The method of claim 1, wherein the methyltransferase with Suv39h-like activity is murine Suv39h2 or human SUV39H2.

4. The method of any one of claims 1 to 3, wherein the substrate is histone H3 or N-terminal fragment thereof that contains the methylation site at lysine 9.

5. The method of claim 4, wherein the histone H3 N-terminal fragment has the amino acid sequence as set forth in SEQ ID NO:7.

6. The method of any one of claims 1 to 5, wherein the methyl donor is radio-labelled methionine or S-adenosyl-L-methionine.

7. The method of any one of claims 1 to 6, wherein the methyl donor carries a chromogenic label and the methyltransferase activity is determined by measuring the change in colour upon transfer of the methyl group to the subtrate.

8. The method of any one of claims 1 to claim 6, wherein the methyl donor carries a radioactive label and the methyltransferase activity is determined by measuring the radioactivity transferred to the substrate upon transfer of the methyl group.

9. The method of any one of claims 1 to 6, wherein the methyltransferase activity is determined immunologically by quantifying the binding of an antibody specific for the methylation site to the substrate.

10. An antibody that specifically recognises methylated lysine 9 in histone H3.

11. A compound identified in a method defined in any one of claims 1 to 9 for the therapy of cancer.


SEQUENCE LISTING

<110> Boehringer Ingelheim International GmbH

<120> Method for identifying compounds altering higher-order chromatin
      dependent chromosome stability

<130> Case 14/055

<140>
<141>

<160> 9

<170> PatentIn Ver. 2.1

<210> 1
<211> 1452
<212> DNA
<213> Mus musculus

<220>
<221> 3'UTR
<222> (1)..(18)

<220>
<221> CDS
<222> (19)..(1452)

<400> 1

gaatgaaagc tccgcaag atg gcg acg gcc agg gcc aag gca cgg ggc agt    51
                    Met Ala Thr Ala Arg Ala Lys Ala Arg Gly Ser
                      1               5                  10

gag gca gga gcg cgg tgt cac cgg gct cca ggt ccg ccc ccg agg ccc    99
Glu Ala Gly Ala Arg Cys His Arg Ala Pro Gly Pro Pro Pro Arg Pro
             15                  20                  25

aag gcc agg cga acg gcg aga cgc cgc cgc gcg gag acc ctg acg gcg    147
Lys Ala Arg Arg Thr Ala Arg Arg Arg Arg Ala Glu Thr Leu Thr Ala
         30                  35                  40

cga cgc tcg cgg ccg tct gcg ggc gag agg cgc gcc ggc tcc cag cga    195
Arg Arg Ser Arg Pro Ser Ala Gly Glu Arg Arg Ala Gly Ser Gln Arg
     45                  50                  55

gcg tgg tcc gga gct ccg cgg gcc gcg gtc ttt ggc gac gag tgt gca    243
Ala Trp Ser Gly Ala Pro Arg Ala Ala Val Phe Gly Asp Glu Cys Ala
 60                  65                  70                  75

cga ggt gcc tta ttc aag gcc tgg tgt gtg cct tgc cta gtt tca ctt    291
Arg Gly Ala Leu Phe Lys Ala Trp Cys Val Pro Cys Leu Val Ser Leu
                 80                  85                  90

gat act ctc cag gaa tta tgt aga aaa gaa aag ctc aca tgt aaa tcg     339
Asp Thr Leu Gln Glu Leu Cys Arg Lys Glu Lys Leu Thr Cys Lys Ser
                95                  100                 105

att gga atc acc aaa agg aat cta aac aat tat gag gtg gag tac ttg     387
Ile Gly Ile Thr Lys Arg Asn Leu Asn Asn Tyr Glu Val Glu Tyr Leu
        110                 115                 120

tgt gac tac aag gta gca aag ggt gtg gaa tat tat ctt gta aaa tgg     435
Cys Asp Tyr Lys Val Ala Lys Gly Val Glu Tyr Tyr Leu Val Lys Trp
    125                 130                 135

aaa gga tgg cca gat tct aca aac acc tgg gag ccc ttg aga aac ctc     483
Lys Gly Trp Pro Asp Ser Thr Asn Thr Trp Glu Pro Leu Arg Asn Leu
140                 145                 150                 155

agg tgt cca cag ctc ctg cgg cag ttc tct gat gac aag aag act tac     531
Arg Cys Pro Gln Leu Leu Arg Gln Phe Ser Asp Asp Lys Lys Thr Tyr
                160                 165                 170

tta gct cag gaa agg aaa tgc aag gct gtc aat tca aaa tcc ttg caa     579
Leu Ala Gln Glu Arg Lys Cys Lys Ala Val Asn Ser Lys Ser Leu Gln
            175                 180                 185

cct gca att gct gag tat att gta cag aaa gct aag caa aga ata gct     627
Pro Ala Ile Ala Glu Tyr Ile Val Gln Lys Ala Lys Gln Arg Ile Ala
        190                 195                 200

ctg cag aga tgg caa gat tac ctc aac aga aga aag aac cat aag ggg     675
Leu Gln Arg Trp Gln Asp Tyr Leu Asn Arg Arg Lys Asn His Lys Gly
    205                 210                 215

atg ata ttt gtt gaa aac act gtt gac ttg gag ggc cca cct tta gac     723
Met Ile Phe Val Glu Asn Thr Val Asp Leu Glu Gly Pro Pro Leu Asp
220                 225                 230                 235

ttc tac tac att aac gag tac agg cca gct ccc ggg atc agc ata aac     771
Phe Tyr Tyr Ile Asn Glu Tyr Arg Pro Ala Pro Gly Ile Ser Ile Asn
                240                 245                 250

agt gaa gcc acc ttt gga tgt tca tgt aca gac tgc ttc ttt gac aag     819
Ser Glu Ala Thr Phe Gly Cys Ser Cys Thr Asp Cys Phe Phe Asp Lys
            255                 260                 265

tgt tgt cct gct gaa gct gga gtt gtg ttg gct tat aat aag aag caa     867
Cys Cys Pro Ala Glu Ala Gly Val Val Leu Ala Tyr Asn Lys Lys Gln
        270                 275                 280

caa att aaa atc caa cca ggc act ccc atc tac gaa tgc aac tca agg     915
Gln Ile Lys Ile Gln Pro Gly Thr Pro Ile Tyr Glu Cys Asn Ser Arg
    285                 290                 295

tgt cga tgt gga cct gaa tgt ccc aat agg att gta caa aaa ggc aca     963
Cys Arg Cys Gly Pro Glu Cys Pro Asn Arg Ile Val Gln Lys Gly Thr
300                 305                 310                 315

caa tat tca ctg tgc atc ttt aaa act agc aat ggc tgt ggt tgg ggt    1011
Gln Tyr Ser Leu Cys Ile Phe Lys Thr Ser Asn Gly Cys Gly Trp Gly
                320                 325                 330

gta aaa acc ctt gtg aag att aaa aga atg agt ttt gtc atg gaa tat    1059
Val Lys Thr Leu Val Lys Ile Lys Arg Met Ser Phe Val Met Glu Tyr
            335                 340                 345

gtt gga gag gtg atc aca agt gaa gag gcc gag aga cgg gga cag ttc    1107
Val Gly Glu Val Ile Thr Ser Glu Glu Ala Glu Arg Arg Gly Gln Phe
        350                 355                 360

tat gac aac aaa ggg atc acc tac ctc ttt gac ctg gac tac gag tct    1155
Tyr Asp Asn Lys Gly Ile Thr Tyr Leu Phe Asp Leu Asp Tyr Glu Ser
    365                 370                 375

gat gag ttc aca gtg gat gca gct cga tat gga aac gta tcc cat ttt    1203
Asp Glu Phe Thr Val Asp Ala Ala Arg Tyr Gly Asn Val Ser His Phe
380                 385                 390                 395

gtg aat cat agt tgt gac cca aat ctt cag gtg ttt agt gtt ttc atc    1251
Val Asn His Ser Cys Asp Pro Asn Leu Gln Val Phe Ser Val Phe Ile
                400                 405                 410

gat aac ctt gat act cgg ctg ccc agg ata gca ttg ttc tct aca aga    1299
Asp Asn Leu Asp Thr Arg Leu Pro Arg Ile Ala Leu Phe Ser Thr Arg
            415                 420                 425

acc ata aac gct gga gaa gag ctg act ttt gac tat caa atg aaa ggt    1347
Thr Ile Asn Ala Gly Glu Glu Leu Thr Phe Asp Tyr Gln Met Lys Gly
        430                 435                 440

tct gga gaa gca tct tca gac tcc att gac cac agc cct gcc aaa aaa    1395
Ser Gly Glu Ala Ser Ser Asp Ser Ile Asp His Ser Pro Ala Lys Lys
    445                 450                 455

agg gtc aga acc caa tgt aaa tgt gga gcc gag act tgc aga ggt tac    1443
Arg Val Arg Thr Gln Cys Lys Cys Gly Ala Glu Thr Cys Arg Gly Tyr
460                 465                 470                 475

ctc aac tga                                                        1452
Leu Asn

<210> 2
<211> 477
<212> PRT
<213> Mus musculus

<400> 2

Met Ala Thr Ala Arg Ala Lys Ala Arg Gly Ser Glu Ala Gly Ala Arg
  1               5                  10                  15

Cys His Arg Ala Pro Gly Pro Pro Pro Arg Pro Lys Ala Arg Arg Thr
             20                  25                  30

Ala Arg Arg Arg Arg Ala Glu Thr Leu Thr Ala Arg Arg Ser Arg Pro
        35                  40                  45

Ser Ala Gly Glu Arg Arg Ala Gly Ser Gln Arg Ala Trp Ser Gly Ala
    50                  55                  60

Pro Arg Ala Ala Val Phe Gly Asp Glu Cys Ala Arg Gly Ala Leu Phe
65                  70                  75                  80

Lys Ala Trp Cys Val Pro Cys Leu Val Ser Leu Asp Thr Leu Gln Glu
                85                  90                  95

Leu Cys Arg Lys Glu Lys Leu Thr Cys Lys Ser Ile Gly Ile Thr Lys
            100                 105                 110

Arg Asn Leu Asn Asn Tyr Glu Val Glu Tyr Leu Cys Asp Tyr Lys Val
        115                 120                 125

Ala Lys Gly Val Glu Tyr Tyr Leu Val Lys Trp Lys Gly Trp Pro Asp
    130                 135                 140

Ser Thr Asn Thr Trp Glu Pro Leu Arg Asn Leu Arg Cys Pro Gln Leu
145                 150                 155                 160

Leu Arg Gln Phe Ser Asp Asp Lys Lys Thr Tyr Leu Ala Gln Glu Arg
                165                 170                 175

Lys Cys Lys Ala Val Asn Ser Lys Ser Leu Gln Pro Ala Ile Ala Glu
            180                 185                 190

Tyr Ile Val Gln Lys Ala Lys Gln Arg Ile Ala Leu Gln Arg Trp Gln
        195                 200                 205

Asp Tyr Leu Asn Arg Arg Lys Asn His Lys Gly Met Ile Phe Val Glu
    210                 215                 220

Asn Thr Val Asp Leu Glu Gly Pro Pro Leu Asp Phe Tyr Tyr Ile Asn
225                 230                 235                 240

Glu Tyr Arg Pro Ala Pro Gly Ile Ser Ile Asn Ser Glu Ala Thr Phe
                245                 250                 255

Gly Cys Ser Cys Thr Asp Cys Phe Phe Asp Lys Cys Cys Pro Ala Glu
            260                 265                 270

Ala Gly Val Val Leu Ala Tyr Asn Lys Lys Gln Gln Ile Lys Ile Gln
        275                 280                 285

Pro Gly Thr Pro Ile Tyr Glu Cys Asn Ser Arg Cys Arg Cys Gly Pro
    290                 295                 300

Glu Cys Pro Asn Arg Ile Val Gln Lys Gly Thr Gln Tyr Ser Leu Cys
305                 310                 315                 320

Ile Phe Lys Thr Ser Asn Gly Cys Gly Trp Gly Val Lys Thr Leu Val
                325                 330                 335

Lys Ile Lys Arg Met Ser Phe Val Met Glu Tyr Val Gly Glu Val Ile
            340                 345                 350

Thr Ser Glu Glu Ala Glu Arg Arg Gly Gln Phe Tyr Asp Asn Lys Gly
        355                 360                 365

Ile Thr Tyr Leu Phe Asp Leu Asp Tyr Glu Ser Asp Glu Phe Thr Val
    370                 375                 380

Asp Ala Ala Arg Tyr Gly Asn Val Ser His Phe Val Asn His Ser Cys
385                 390                 395                 400

Asp Pro Asn Leu Gln Val Phe Ser Val Phe Ile Asp Asn Leu Asp Thr
                405                 410                 415

Arg Leu Pro Arg Ile Ala Leu Phe Ser Thr Arg Thr Ile Asn Ala Gly
            420                 425                 430

Glu Glu Leu Thr Phe Asp Tyr Gln Met Lys Gly Ser Gly Glu Ala Ser
        435                 440                 445

Ser Asp Ser Ile Asp His Ser Pro Ala Lys Lys Arg Val Arg Thr Gln
    450                 455                 460

Cys Lys Cys Gly Ala Glu Thr Cys Arg Gly Tyr Leu Asn
465                 470                 475

<210> 3
<211> 543
<212> DNA
<213> Homo sapiens

<220>
<221> misc_feature
<222> (1)..(543)
<223> EST Acc. No.173625

<400> 3

ggccatgtgg ttgancccct ggntttaccn nnccntggnn ggnnttgann ccccttagat 60
tatagtccag aatcattgtt gtcatataac tgccctcatc tttcagcttc gtcacttgtg 120
attacctttc caacttattc catgacaaaa cttattcttt taatcttcac atgggttttt 180
acaccccagc catggtcatt gatactgtga aagatgcaaa gtgaattact gtgtgccttt 240
ttgtacaatc ctattggtac agtgaggtcc acattgacag attgagatgc atttatagat 300
gggagtaaca ggtgggattt taatttgttg gtttttacta taagccaaaa gaattccagc 360
ttcaccaaga caacattttt catagaagca atctgtgcat gaacaacaaa aggtagcttc 420
atttactaag ctgattccag gagctggttt gtattcatca atatagcaga agtctgaagg 480
tgggccttct aagtgaaccc tattntcaac aaatatcact cctttattat tctgtcttct 540
gcg                                                                543

<210> 4
<211> 579
<212> DNA
<213> Homo sapiens

<220>
<221> misc_feature
<222> (1)..(579)
<223> EST Acc. No. AQ494637

<400> 4

gcttctcata catgatacgt gttcngctct gnngtntnng tttangaata cntaaaanaa 60
aaggnagggg ngncntttga ttcgtgtgat tccatagatg cactcatatg gaactgtatt 120
tcattntgtg aatcatagta gtgacccaaa tcttcatatg ttctatgntn tcactgataa 180
cttgacactg gccttcccta tatagctctg tgttccatga gaactataaa tgctggagaa 240
gagttgattt ttgacaatca aacaaaaagt tctggggata tatcttcaga gtttattgac 300
cacagctcag ccaaaaagag ggtcagaact gtatgtaaat gtggagctgt gacttgcaga 360
ggttgcctca aatgaatttt caggaaatag aaatgatgat aattggtagt tgtttctttt 420
ttctaatgtt atcattctaa aaataagtat ttggaactct cttttcatat tatcaagatt 480
attactatgt taaattgaca tncatggttc aaggcattta ccanatgcat tactgatgcc 540
tcttgagaga gggccactgt gttgcataga ctgatctga 579

<210> 5
<211> 565
<212> DNA
<213> Homo sapiens

<220>
<221> misc_feature
<222> (1)..(565)
<223> EST Acc. No. AQ691972

<400> 5

agaggatgag catggatcnt cgctatagca aaccacanat anaatcccac ctgttactcc 60
catctataaa tgcatctcaa tctgtcaatg tggaccttac tgtaccaata ggattgtaca 120
aaaaggcaca cagtaattca ctttgcatct ttcacagtat caatgaccat ggctggggtg 180
taaaaaccca tgtgaagatt aaaagaataa gttttgtcat ggaataagtt ggaaaggtaa 240
tcacaagtga cgaagctgaa agatgagggc agttatatga caacaaatga tctggactat 300
gaatctgatg aattcacaga ggatgcagct caatatggaa ctgtatttca ttntgtgaat 360
cataagtagt gacccaaact tcatatgttc aatgttntca ttgataactt gacactggcc 420
tttccttaat agctctgtgt tccatgagaa ctataaatgc tggagaagaa gtgatttttg 480
acatcaacaa aagttctggg attatcttca aagttattgc cacagttacc aaaagaaggc 540
aaactgttgt aatgtgagct gtact 565

<210> 6
<211> 535
<212> DNA
<213> Homo sapiens

<220>
<221> misc_feature
<222> (1)..(535)
<223> EST Acc. No. AQ554070

<400> 6

tcagactcat agtccagatc aaagagattc tgtgattccc ttgttgtcat agaactgtcc 60
tcgtctttca gcttcttcac ttgtgattac ctaaacagaa aaaactgtaa gtatattacg 120
tagctactga accaaagaag cattcatcta cctatctact aatatgcgaa tacctacaaa 180
tatttaaaaa gtaagaaatt caggtgtcat caaagcaaac attcacacaa actaagactc 240
agatgcaaag aggtgggaaa atgaggggaa gaaaaatgat aatgcaaaag actgatgacc 300
tttttttttt aaacagggtc tcactctgtc actcaggcta gaatgcggtg gtgccatcat 360
gactccctgt atcctttaac tcctgggatc aagcgatctt cctgcctcag cctcctgact 420
agctggatca caggtgcata ccgccatgcc cagctaatga tttagttttt atagagatgt 480
ggggtctcac tatgttgccc acactggtct ggaactcctg ggctcaagtg agcct 535

<210> 7
<211> 20
<212> PRT
<213> Homo sapiens

<400> 7

Ala Arg Thr Lys Gln Thr Ala Arg Lys Ser Thr Gly Gly Lys Ala Pro
1               5                   10                  15

Arg Lys Gln Leu
            20

210> 8
<211> 20
<212> PRT
<213> Homo sapiens

<400> 8

Met Gly Pro Arg Arg Arg Ser Arg Lys Pro Glu Ala Pro Arg Arg Arg Ser Pro Ser Pro
1               5                   10                  15                  20

210> 9
<211> 22
<212> PRT
<213> Rattus rattus

<400> 9

Met Glu Thr Ser Ser Arg Gly Gly Lys Lys Lys Ser Thr Lys Thr Ser Arg Ser Ala Lys
1               5                   10                  15                  20

Ala Gly
22